# EUROPÄISCHE PATENTSCHRIFT

(11) **EP 4 408 345 B1**
(45) Veröffentlichungstag und Bekanntmachung des Hinweises auf die Patenterteilung: **29.01.2025**
(21) Anmeldenummer: 22786014.5
(22) Anmeldetag: 16.09.2022
(51) Int. Cl.: A61F 2/16

(54) **AKKOMMODATIVE INTRAOKULARLINSE ZUM ERZEUGEN EINER RÜCKSTELLKRAFT**
ACCOMMODATIVE INTRAOCULAR LENS FOR PRODUCING A RESTORING FORCE
LENTILLE INTRAOCULAIRE D'ACCOMMODATION POUR GÉNÉRER UNE FORCE DE RAPPEL

(30) Priorität: 29.09.2021 DE 102021125295
(43) Veröffentlichungstag der Anmeldung: 07.08.2024
(73) Patentinhaber: Carl Zeiss Meditec AG, 07745 Jena (DE)
(72) Erfinder: SCHREIBER, Benjamin, 73447 Oberkochen (DE); WOLF, Uwe, 73447 Oberkochen (DE); WOLFSTEIN, André, 73447 Oberkochen (DE); BUCHHEISTER, Jan, 73447 Oberkochen (DE); SRBINOSKA, Hristina, 73447 Oberkochen (DE); NICOLI, Francesca, 73447 Oberkochen (DE)
(74) Vertreter: PATERIS Patentanwälte PartmbB
(86) Internationale Anmeldenummer: PCT/EP2022/075815
(87) Internationale Veröffentlichungsnummer: WO 2023/052154

(56) Entgegenhaltungen:
- EP-A1- 3 829 488
- DE-B3- 102020 123 518
- DE-T2- 60 307 816
- US-A1- 2018 161 151
- US-A1- 2020 046 490

## Beschreibung

Die Erfindung betrifft eine akkommodative Intraokularlinse zum Erzeugen einer Rückstellkraft. DE 10 2020 123 518 B3 offenbart eine akkommodative Intraokularlinse. US 2020/0046490 A1 offenbart eine dualoptische, krümmungsändernde akkommodative Intraokularlinse. US 2018/0161151 A1 offenbart verzweigte haptische Ausrichtungsaktuatoren für akkommodative Intraokularlinse. DE 603 07 816 T2 offenbart eine akkommodative Intraokularlinse. WO 2020/025325 A1 offenbart eine akkommodative Intraokularlinse.

Mit einer natürlichen Augenlinse ist es möglich, in der Ferne und in der Nähe Gegenstände scharf zu sehen. Ermöglicht wird dies dadurch, dass die Augenlinse in ihrer Form und damit die Brechkraft verändert werden kann. Die Augenlinse ist in einem Kapselsack enthalten, der an Zonulafasern aufgehängt ist, welche wiederum mit einem Ziliarmuskel verbunden sind. Wenn der Ziliarmuskel sich entspannt, werden die Zonulafasern gestrafft, wodurch der Kapselsack gestreckt wird. Die Formänderung des Kapselsackes bewirkt bei einer weichen Augenlinse, dass diese sich ebenfalls in ihrer Form verändert. Mit zunehmender Streckung des Kapselsackes wird die Augenlinse zunehmend abgeflacht. Dadurch ändert sich die Brechkraft der Augenlinse. Eine abgeflachte Augenlinse führt zu einer geringeren Brechkraft, sodass eine scharfe Fernsicht ermöglicht wird. Dieser Vorgang ist reversibel, sodass bei einem angespannten Ziliarmuskel die Zonulafasern erschlaffen und der Kapselsack weniger stark gestreckt ist. Damit nimmt die Augenlinse eine Form an, die stärker gewölbt ist, sodass eine höhere Refraktion erreicht wird. Dadurch ist es möglich, Gegenstände in der Nähe scharf zu sehen. Diese Variation in der Schärfenebene wird als Akkommodation bezeichnet.

Es ist normal, dass mit zunehmendem Alter die Augenlinse an Elastizität verliert. Die Augenlinse ist dann weniger in der Lage, ihre Form als Reaktion auf eine Kontraktion des Ziliarmuskels zu ändern. Dies bewirkt, dass ein Fokussieren auf nahe Gegenstände immer schwerer gelingt. Dieser Zustand wird mit Presbyopie bezeichnet. Durch das Tragen einer Brille oder einer Kontaktlinse ist es möglich, die fehlende Brechkraft zu kompensieren. Mit zunehmendem Alter wird die Augenlinse jedoch zunehmend inelastischer bis hart und kann zudem eintrüben. In der Medizin wird eine solcher Zustand der Augenlinse mit Katarakt oder "grauer Star" bezeichnet. Ein Brillenglas kann die Folgen einer Eintrübung der Augenlinse nicht kompensieren, sodass es üblich geworden ist, die eingetrübte Augenlinse chirurgisch zu entfernen. Dazu wird z.B. eine mit Ultraschall schwingende Nadel in das Auge eingeführt und die harte und eingetrübte Augenlinse in kleine Partikel zertrümmert. Dieser Vorgang wird als Phakoemulsifikation bezeichnet. Die Partikel werden nach einer solchen Phakoemulsifikation abgesaugt, bis der Kapselsack von der natürlichen Augenlinse befreit ist. Um wieder ein gutes Sehen zu ermöglichen, wird anschließend eine künstliche Augenlinse in den Kapselsack implantiert. Diese künstliche Augenlinse wird als Intraokularlinse bezeichnet.

Die künstliche Augenlinse ist üblicherweise eine Linse mit einem einzigen Brennpunkt (monofokal), sodass ein Patient nach einer Implantation einer künstlichen Augenlinse eine Brille oder eine Kontaktlinse benötigt, um sowohl in der Ferne als auch in der Nähe scharf zu sehen. Es gibt jedoch auch Überlegungen, die künstliche Augenlinse so zu gestalten, dass eine Akkommodation mit einer sich verändernden Schärfenebene möglich ist. Eine solche künstliche Augenlinse wird auch als akkommodative Intraokularlinse bezeichnet. Das Anspannen oder Entspannen eines Ziliarmuskels soll es ermöglichen, die Form der Intraokularlinse und damit deren Brechkraft zu verändern. Wünschenswert wäre es, eine künstliche Augenlinse zu schaffen, die auch ähnliche mechanische Eigenschaften mit der natürlichen Augenlinse hat.

US 2010 / 0 179 653 A1 offenbart eine akkommodierende Intraokularlinse. US 2014 / 0 368 789 A1 offenbart ein adaptives Linsensystem.

Aufgabe der Erfindung ist es daher, eine akkommodative Intraokularlinse zu schaffen, die ähnliche mechanische Eigenschaften wie eine natürliche Augenlinse hat.

Eine erste erfindungsgemäße akkommodative Intraokularlinse zum Einbringen in den Kapselsack eines Auges weist einen ersten Linsenteil und einen zweiten Linsenteil auf. Der erste Linsenteil weist ein für Licht transparentes Linsenbauteil auf, das eine distale Oberfläche des Linsenbauteils und eine proximale Oberfläche des Linsenbauteils aufweist. Zudem weist der erste Linsenteil eine für das Licht transparente erste Membran, die benachbart zu der distalen Oberfläche des Linsenbauteils angeordnet ist und zusammen mit dem Linsenbauteil einen Hohlraum begrenzt. Des Weiteren weist der erste Linsenteil eine optische Achse auf. Der zweite Linsenteil lässt sich lösbar mit dem ersten Linsenteil koppeln, wodurch die Intraokularlinse in einen Kopplungszustand bringbar ist, in dem der zweite Linsenteil an einer distalen Seite des ersten Linsenteils angeordnet ist und eingerichtet ist, durch ein Längsverlagern des zweiten Linsenteils parallel zu der optischen Achse die erste Membran zu verformen. Der zweite Linsenteil weist ein erstes Bauteil, das ein distales Ende des zweiten Linsenteils bildet und ein Axialdurchgangsloch des ersten Bauteils auf, und ein zweites Bauteil auf, das eingerichtet ist, die erste Membran zu verformen, und ein Axialdurchgangsloch des zweiten Bauteils aufweist. Die optische Achse erstreckt sich durch das Axialdurchgangsloch des ersten Bauteils und durch das Axialdurchgangsloch des zweiten Bauteils. Das erste Bauteil und das zweite Bauteil sind an ihren Enden, die in dem Kopplungszustand in einer Radialrichtung bezüglich der optischen Achse außen angeordnet sind, fest miteinander verbunden, wobei das erste Bauteil und das zweite Bauteil eingerichtet sind, bei einer zunehmenden Streckung des Kapselsacks zueinander hin zu verlagern und dadurch eine Rückstellkraft zu erzeugen.

Eine zweite erfindungsgemäße akkommodative Intraokularlinse zum Einbringen in den Kapselsack eines Auges weist einen ersten Linsenteil und einen zweiten Linsenteil auf. Der erste Linsenteil weist ein für Licht transparentes Linsenbauteil auf, das eine distale Oberfläche des Linsenbauteils und eine proximale Oberfläche des Linsenbauteils aufweist. Zudem weist der erste Linsenteil eine für das Licht transparente erste Membran auf, die benachbart zu der distalen Oberfläche des Linsenbauteils angeordnet ist und zusammen mit dem Linsenbauteil einen Hohlraum begrenzt. Des Weiteren weist der erste Linsenteil eine optische Achse auf. Der zweite Linsenteil lässt sich lösbar mit dem ersten Linsenteil koppeln, wodurch die Intraokularlinse in einen Kopplungszustand bringbar ist, in dem der zweite Linsenteil an einer distalen Seite des ersten Linsenteils angeordnet ist und eingerichtet ist, durch ein Längsverlagern des zweiten Linsenteils parallel zu der optischen Achse die erste Membran zu verformen. Der zweite Linsenteil weist ein erstes Bauteil, das ein distales Ende des zweiten Linsenteils bildet und ein Axialdurchgangsloch des ersten Bauteils aufweist, und ein zweites Bauteil auf, das sich in Richtung der optischen Achse mindestens bis zu dem Linsenbauteil erstreckt und ein Axialdurchgangsloch des zweiten Bauteils aufweist. Die optische Achse erstreckt sich durch das Axialdurchgangsloch des ersten Bauteils und durch das Axialdurchgangsloch des zweiten Bauteils. Das erste Bauteil und das zweite Bauteil sind an ihren Enden, die in dem Kopplungszustand in einer Radialrichtung bezüglich der optischen Achse außen angeordnet sind, fest miteinander verbunden, wobei das erste Bauteil und das zweite Bauteil eingerichtet sind, bei einer zunehmenden Streckung des Kapselsacks zueinander hin zu verlagern und dadurch eine Rückstellkraft zu erzeugen.

Die Anordnung aus dem Linsenbauteil, dem Hohlraum und der ersten Membran ist eingerichtet, eine optische Abbildung durchzuführen, wodurch der erste Linsenteil die optische Achse aufweist. Um die Intraokularlinse in den Kapselsack eines Auges einzusetzen, wird ein Schnitt in den Kapselsack eingebracht. Es werden zuerst der erste Linsenteil und anschließend der zweite Linsenteil via den Schnitt in den Kapselsack eingesetzt. Der zweite Linsenteil wird so in den Kapselsack angeordnet, dass die Intraokularlinse in den Kopplungszustand gelangt. Weil durch das Längsverlagern des zweiten Linsenteils die Membran verformt, erfährt die Membran eine Änderung ihres Krümmungsradius. Indem die Membran ihren Krümmungsradius ändert, ändert die Membran ihre Refraktion, sodass eine Akkommodation des Auges auf Gegenstände in der Ferne oder in der Nähe erreichbar ist.

Die Rückstellkraft entsteht, weil bei einem Entspannen eines Ziliarmuskels der Kapselsack eine zunehmende Streckung erfährt. Dadurch drückt ein vorderer Bereich des Kapselsacks auf das erste Bauteil. Bei der ersten erfindungsgemäßen Intraokularlinse drückt ein hinterer Bereich des Kapselsacks via den ersten Linsenteil auf das zweite Bauteil. Bei der zweiten erfindungsgemäßen Intraokularlinse kontaktiert das zweite Bauteil den hinteren Bereich des Kapselsacks, so dass der hintere Bereich des Kapselsacks unmittelbar auf das zweite Bauteil drückt. Dadurch verlagern das erste Bauteil und das zweite Bauteil in Richtung zueinander hin, wodurch die Rückstellkraft entsteht. Die Rückstellkraft führt dazu, dass der Kapselsack wieder weniger gestreckt wird, wenn der Ziliarmuskel nach dem Entspannen wieder angespannt wird. Dadurch hat die Intraokularlinse ähnliche mechanische Eigenschaften wie eine natürliche Augenlinse.

Für die zweite erfindungsgemäße Intraokularlinse ist es bevorzugt, dass das erste Bauteil einen zylinderförmigen Abschnitt aufweist, der in dem Kopplungszustand das in der Radialrichtung innenliegende Ende des ersten Bauteils bildet und der den ersten Linsenteil kontaktiert.

Für die zweite erfindungsgemäße Intraokularlinse ist es bevorzugt, dass das zweite Bauteil sich in der Axialrichtung mindestens bis zu einer proximalen Seite des ersten Linsenteils erstreckt. Zudem ist es für die zweite erfindungsgemäße Intraokularlinse bevorzugt, dass eine proximale Oberfläche des zweiten Bauteils konvex gekrümmt ausgeführt ist. Dadurch kann sich der hintere Bereich des Kapselsacks gut an das zweite Bauteil anschmiegen, wodurch eine Verletzung des Gewebes des hinteren Bereichs des Kapselsacks durch das zweite Bauteil vermieden werden kann.

Für beide erfindungsgemäßen Intraokularlinse ist es bevorzugt, dass das erste Bauteil und/oder das zweite Bauteil in der Radialrichtung eine maximale Erstreckung von mindestens 8,5 mm haben. Dadurch ist es möglich, den Kapselsack sowohl in einem nicht gestreckten Zustand als auch in einem gestreckten Zustand seitlich aufzuspannen und offen zu halten. Dadurch kann das Gewebe des Kapselsacks vor einer Verletzung bewahrt werden. Die maximale Erstreckung kann beispielsweise maximal 10,0 mm betragen.

Es ist für beide erfindungsgemäße Intraokularlinsen bevorzugt, dass das Linsenbauteil eine zweite Membran oder ein Optikkörper ist.

Der Elastizitätsmodul des ersten Bauteils und des zweiten Bauteils beträgt für beide erfindungsgemäßen Intraokularlinsen bevorzugt von 1,5 MPa bis 5,0 MPa. Der Elastizitätsmodul der ersten Membran und/oder der zweiten Membran beträgt für beide erfindungsgemäßen Intraokularlinse bevorzugt maximal 3 MPa, insbesondere von 1,5 MPa bis 2,0 MPa.

Es ist für beide erfindungsgemäßen Intraokularlinsen bevorzugt, dass der zweite Linsenteil eine weitere Linse aufweist. Dabei ist besonders bevorzugt, dass eine optische Achse der weiteren Linse mit der optischen Achse des ersten Linsenteils im Wesentlichen zusammenfällt. Die weitere Linse kann beispielswese torisch sein. Dadurch kann vorteilhaft eine Hornhautverkrümmung korrigiert werden. Es zudem denkbar, dass die weitere Linse nicht akkommodativ ausgeführt ist.

Eine distale Oberfläche des ersten Bauteils ist für beide erfindungsgemäßen Intraokularlinsen bevorzugt konvex gekrümmt ausgeführt. Dadurch hat die den Kapselsack kontaktierende Oberfläche des ersten Bauteils die Form des Kapselsacks. Dadurch kann sich der vordere Bereich des Kapselsacks besonders gut an das erste Bauteil anschmiegen, wodurch eine Verletzung des Gewebes des Kapselsacks durch das erste Bauteil vermieden werden kann.

Das erste Bauteil und/oder das zweite Bauteil weist für beide erfindungsgemäßen Intraokularlinsen bevorzugt eine Mehrzahl an Radialdurchgangslöchern auf, via die eine Flüssigkeit von einer in der Radialrichtung innenliegenden Seite des zweiten Linsenteils zu einer in der Radialrichtung außenliegenden Seite des zweiten Linsenteils strömen kann. Indem via die Radialdurchgangslöcher in dem Kapselsack mehr Strömung erfolgt als ohne die Radialdurchgangslöcher erfolgt, kann dadurch die Wahrscheinlichkeit eines Auftretens eines Nachstars verringert werden.

Es ist für beide erfindungsgemäßen Intraokularlinsen bevorzugt, dass der zweite Linsenteil eine Mehrzahl an Füßen aufweist, die in einem axialen Bereich das in der Radialrichtung außenliegende Ende des zweiten Linsenteils bilden, in einer proximalen Richtung von dem verbliebenen zweiten Linsenteil abstehen und in einer Umfangsrichtung bezüglich der optischen Achse benachbart zueinander angeordnet sind. Mittels der Füße kann ein besonders großer Bereich des Kapselsacks aufgespannt werden und dadurch, dass die Füße beabstandet voneinander angeordnet sind, ist eine Strömung zwischen den Füßen möglich, wodurch die Wahrscheinlichkeit eines Auftretens eines Nachstars verringert ist. Die Füße sind besonders bevorzugt eingerichtet, in dem Kopplungszustand den ersten Linsenteil, insbesondere das Linsenbauteil, zu halten und somit den ersten Linsenteil bezüglich des zweiten Linsenteils zu zentrieren. Dazu können die Füße beispielsweise rotationssymmetrisch bezüglich der optischen Achse ausgebildet sein.

Im Folgenden wird anhand der beigefügten schematischen Zeichnungen die Erfindung näher erläutert. Es zeigen
Figur 1 einen Längsschnitt durch eine erste Ausführungsform der erfindungsgemäßen akkommodativen Intraokularlinse in einem Kapselsack, der sich in einem nicht gestreckten Zustand befindet,
Figur 2 einen Längsschnitt durch die Intraokularlinse gemäß Figur 1, wobei der Kapselsack sich in einem gestreckten Zustand befindet,
Figur 3 einen Längsschnitt durch eine zweite Ausführungsform der erfindungsgemäßen akkommodativen Intraokularlinse und
Figur 4 einen Längsschnitt durch eine dritte Ausführungsform der erfindungsgemäßen akkommodativen Intraokularlinse.

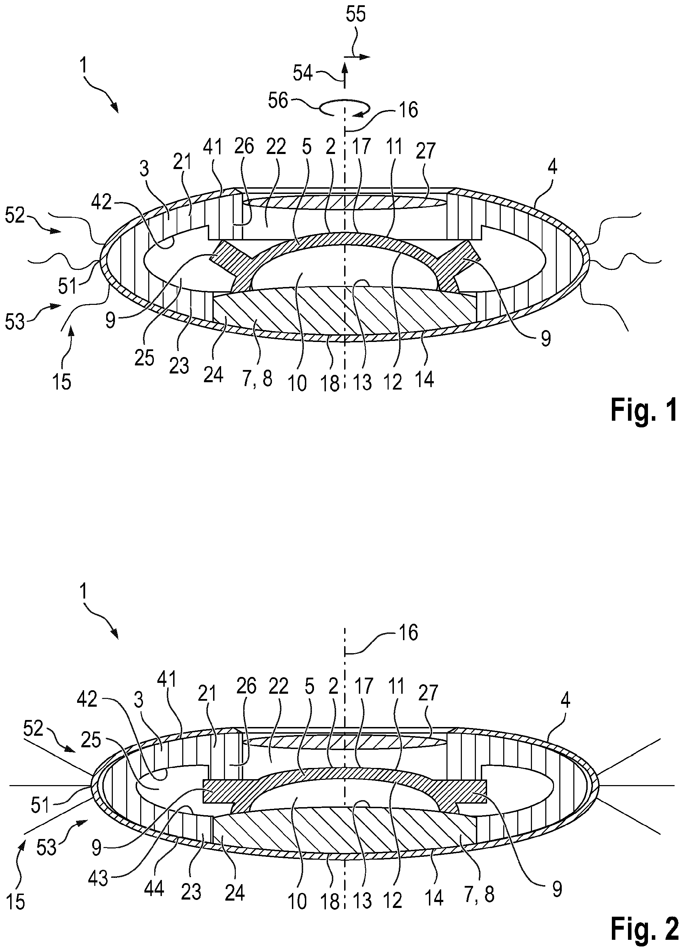

Figuren 1 und 2 zeigen, dass gemäß einer ersten Ausführungsform der akkommodativen Intraokularlinse 1 zum Einbringen in den Kapselsack 4 eines Auges die Intraokularlinse 1 einen ersten Linsenteil 2 und einen zweiten Linsenteil 3 aufweist. Der erste Linsenteil 2 weist ein für Licht transparentes Linsenbauteil 7, das eine distale Oberfläche 13 des Linsenbauteils 7 und eine proximale Oberfläche 14 des Linsenbauteils 7 aufweist, eine für das Licht transparente erste Membran 5, die benachbart zu der distalen Oberfläche 13 des Linsenbauteils 7 angeordnet ist und zusammen mit dem Linsenbauteil 7 einen Hohlraum 10 begrenzt, und eine optische Achse 16 auf. Der zweite Linsenteil 3 lässt sich lösbar mit dem ersten Linsenteil 2 koppeln, wodurch die Intraokularlinse 1 in einen Kopplungszustand bringbar ist, in dem der zweite Linsenteil 3 an einer distalen Seite 17 des ersten Linsenteils 2 angeordnet ist und eingerichtet ist, durch ein Längsverlagern des zweiten Linsenteils 3 parallel zu der optischen Achse 16 die erste Membran 5 zu verformen. Der zweite Linsenteil 3 weist ein erstes Bauteil 21, das ein distales Ende des zweiten Linsenteils 3 bildet und ein Axialdurchgangsloch 22 des ersten Bauteils 21 aufweist, und ein zweites Bauteil 23 auf, das sich in Axialrichtung 54 bezüglich der optischen Achse 15 mindestens bis zu dem Linsenbauteil 7 erstreckt und ein Axialdurchgangsloch 24 des zweiten Bauteils 23 aufweist. Die optische Achse 16 erstreckt sich durch das Axialdurchgangsloch 22 des ersten Bauteils 21 und durch das Axialdurchgangsloch 24 des zweiten Bauteils 23. Das erste Bauteil 21 und das zweite Bauteil 23 sind an ihren Enden, die in dem Kopplungszustand in einer Radialrichtung 55 bezüglich der optischen Achse 16 außen angeordnet sind, fest miteinander verbunden, wobei das erste Bauteil 21 und das zweite Bauteil 23 eingerichtet sind, bei einer zunehmenden Streckung des Kapselsacks 4 zueinander hin zu verlagern und dadurch eine Rückstellkraft zu erzeugen.

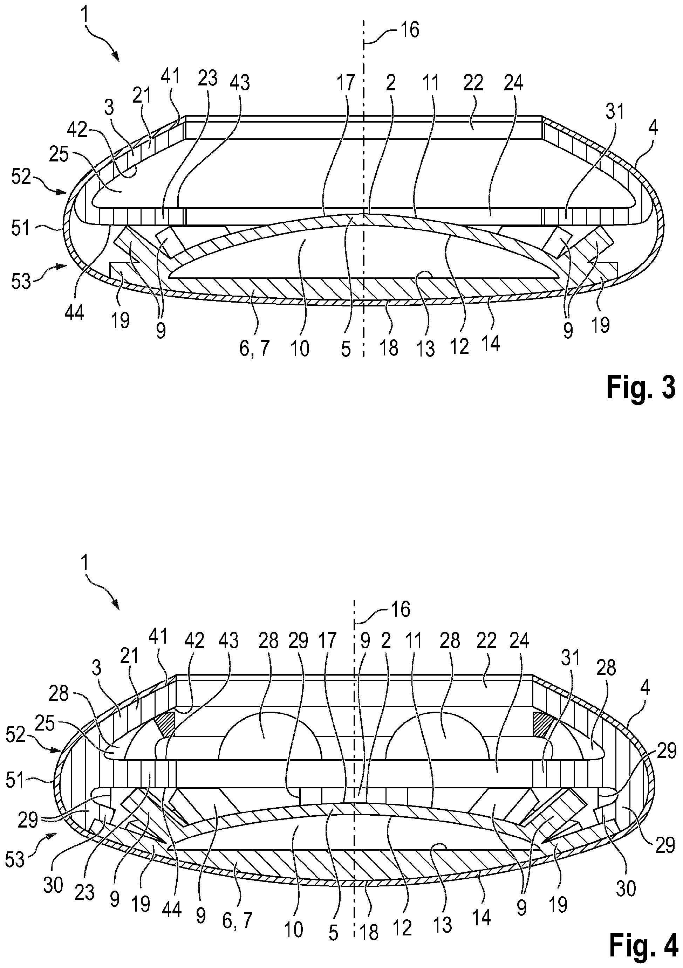

Wie es aus Figuren 3 und 4 ersichtlich ist, weist gemäß einer zweiten und dritten Ausführungsform einer akkommodativen Intraokularlinse 1 zum Einbringen in den Kapselsack 4 eines Auges die Intraokularlinse 1 einen ersten Linsenteil 2 und einen zweiten Linsenteil 3 auf. Der erste Linsenteil 2 weist ein für Licht transparentes Linsenbauteil 7, das eine distale Oberfläche 13 des Linsenbauteils 7 und eine proximale Oberfläche 14 des Linsenbauteils 7 aufweist, eine für das Licht transparente erste Membran 5, die benachbart zu der distalen Oberfläche 13 des Linsenbauteils 7 angeordnet ist und zusammen mit dem Linsenbauteil 7 einen Hohlraum 10 begrenzt, und eine optische Achse 16 auf. Der zweite Linsenteil 3 lässt sich lösbar mit dem ersten Linsenteil 2 koppeln, wodurch die Intraokularlinse 1 in einen Kopplungszustand bringbar ist, in dem der zweite Linsenteil 3 an einer distalen Seite 17 des ersten Linsenteils 2 angeordnet ist und eingerichtet ist, durch ein Längsverlagern des zweiten Linsenteils 3 parallel zu der optischen Achse 16 die erste Membran 5 zu verformen. Der zweite Linsenteil 3 weist ein erstes Bauteil 21, das ein distales Ende des zweiten Linsenteils 3 bildet und ein Axialdurchgangsloch 22 des ersten Bauteils 21 aufweist, und ein zweites Bauteil 23 auf, das eingerichtet ist, die erste Membran 5 zu verformen, und ein Axialdurchgangsloch 24 des zweiten Bauteils 23 aufweist. Die optische Achse 16 erstreckt sich durch das Axialdurchgangsloch 22 des ersten Bauteils 21 und durch das Axialdurchgangsloch 24 des zweiten Bauteils 23. Das erste Bauteil 21 und das zweite Bauteil 23 sind an ihren Enden, die in dem Kopplungszustand in einer Radialrichtung 55 bezüglich der optischen Achse 16 außen angeordnet sind, fest miteinander verbunden, wobei das erste Bauteil 21 und das zweite Bauteil 23 eingerichtet sind, bei einer zunehmenden Streckung des Kapselsacks 4 zueinander hin zu verlagern und dadurch eine Rückstellkraft zu erzeugen.

In Figur 1 ist der Kapselsack 4 in einem nicht gestreckten Zustand dargestellt, in dem an dem Kapselsack 4 befestigte Zonulafasern 15 erschlafft sind. Figur 2 zeigt, dass durch ein Erschlaffen eines Ziliarmuskels (nicht dargestellt) die Zonulafasern 15 gestrafft werden, wodurch der Kapselsack 4 in einen gestreckten Zustand gelangt. Dadurch drückt ein vorderer Bereich 52 des Kapselsacks 4 auf das erste Bauteil 21. Ein hinterer Bereich 53 des Kapselsacks 4 drückt auf das zweite Bauteil 23, wobei gemäß der ersten Ausführungsform der hintere Bereich 53 des Kapselsacks 4 unmittelbar auf das erste Bauteil 21 drückt und gemäß der zweiten Ausführungsform und der dritten Ausführungsform der hintere Bereich 53 mittelbar via den ersten Linsenteil 2 auf das zweite Bauteil 23 drückt. Dadurch verlagern das erste Bauteil 21 und das zweite Bauteil 23 zueinander hin, wodurch die Rückstellkraft erzeugt wird. Erschlaffen die Zonulafasern 15 wieder durch ein Anspannen des Ziliarmuskels, gelangt der Kapselsack 4 aufgrund der Rückstellkraft wieder in den nicht gestreckten Zustand gemäß Figur 1.

Wie es aus Figuren 1 und 2 ersichtlich ist, kann gemäß der ersten Ausführungsform das erste Bauteil 21 einen zylinderförmigen Abschnitt 26 aufweisen, der in dem Kopplungszustand das in der Radialrichtung 55 innenliegende Ende des ersten Bauteils 21 bildet und der den ersten Linsenteil 2 kontaktiert. Der zylinderförmige Abschnitt 26 kann dabei in einer Axialrichtung 54 bezüglich der optischen Achse 16 hin zu dem ersten Linsenteil 2 von dem verbliebenen ersten Bauteil 21 abstehen.

Figuren 1 und 2 zeigen, dass gemäß der ersten Ausführungsform das zweite Bauteil 23 sich in dem Kopplungszustand in der Axialrichtung 54 mindestens bis zu einer proximalen Seite 8 des ersten Linsenteils 2 erstrecken kann. Außerdem ist ersichtlich, dass eine proximale Oberfläche 44 des zweiten Bauteils 23 konvex gekrümmt ausgeführt sein kann.

Gemäß der zweiten Ausführungsform und der dritten Ausführungsform kann das zweite Bauteil 23 einen scheibenförmigen Abschnitt 31 aufweisen, der den ersten Linsenteil 2 kontaktiert und der das Axialdurchgangsloch 24 des zweiten Bauteils 23 begrenzt, vergleiche Figuren 3 und 4. Der scheibenförmige Abschnitt 31 und das erste Bauteil 21 können in dem Kopplungszustand einen Zwischenraum 25 des zweiten Linsenteils 3 in der Axialrichtung 54 begrenzen. Der Zwischenraum 25 kann in der Radialrichtung nach innen offen sein.

Für alle Ausführungsformen gilt, dass das erste Bauteil 21 und/oder das zweite Bauteil 23 sich bis zu einem Äquator 51 des Kapselsacks 4 erstrecken können, wobei der Äquator 51 einen vorderen Bereich 52 des Kapselsacks 4 von einem hinteren Bereich 53 des Kapselsacks trennt. Dazu können das erste Bauteil 21 und/oder das zweite Bauteil 23 in der Radialrichtung 55 eine maximale Erstreckung von mindestens 8,5 mm haben. Die maximale Erstreckung in der Radialrichtung 55 kann beispielsweise maximal 10,0 mm betragen.

Wie es aus Figuren 1 bis 4 ersichtlich ist, kann der erste Linsenteil 2 eine Mehrzahl an Biegeelementen 9 aufweisen, die an der distalen Oberfläche 11 der ersten Membran 5 angeordnet sind, und die in Umfangsrichtung 56 bezüglich der optischen Achse 16 beabstandet voneinander angeordnet sind. Bei der ersten Ausführungsformen stehen die Biegeelemente 9 in Kontakt mit dem ersten Bauteil 21, insbesondere mit dem zylinderförmigen Abschnitt 26. Bei der zweiten und dritten Ausführungsform stehen die Biegeelemente 9 in Kontakt mit dem zweiten Bauteil 23.

Figuren 1 und 2 zeigen, dass das Linsenbauteil 7 beispielsweise ein Optikkörper 8 sein kann. Figuren 3 und 4 zeigen, dass das Linsenbauteil 7 beispielsweise eine zweite Membran 6 sein kann.

Der Elastizitätsmodul des ersten Bauteils 21 und des zweiten Bauteils 23 kann von 1,5 MPa bis 5,0 Pa betragen. Der Elastizitätsmodul der ersten Membran 5 und/oder der zweiten Membran 6 kann maximal 3 MPa, insbesondere von 1,5 MPa bis 2 MPa, betragen.

Figuren 1 und 2 zeigen, dass der zweite Linsenteil 3 eine weitere Linse 27 aufweisen kann. Die optische Achse der weitere Linse 27 kann dabei in dem Kopplungszustand im Wesentlichen mit der optischen Achse 16 des ersten Linsenteils 2 zusammenfallen. Die weitere Linse 27 kann beispielsweise torisch sein. Dadurch ist es möglich, mittels der weiteren Linse eine Hornhautverkrümmung zu korrigieren. Zudem ist denkbar, dass die weitere Linse 27 nicht akkommodativ ausgeführt ist, d.h. sie kann im Gegensatz zu dem ersten Linsenteil 2 ihre Brechkraft nicht verändern.

Aus Figuren 1 bis 4 ist ersichtlich, dass eine distale Oberfläche 41 des ersten Bauteils 21 konvex gekrümmt ausgeführt sein kann.

Figur 4 zeigt, dass das erste Bauteil 21 und/oder das zweite Bauteil 23 eine Mehrzahl an Radialdurchgangslöchern 28 aufweisen können, via die eine Flüssigkeit von einer in der Radialrichtung 55 innenliegenden Seite des zweiten Linsenteils 3 zu einer in der Radialrichtung 55 außenliegenden Seite des zweiten Linsenteils 3 strömen kann.

Zudem zeigt Figur 4, dass der zweite Linsenteil 3 eine Mehrzahl an Füßen 29 aufweisen kann, die in einem axialen Bereich das in der Radialrichtung 55 außenliegende Ende des zweiten Linsenteils 3 bilden, in einer proximalen Richtung von dem verbliebenen zweiten Linsenteil 3 abstehen und in einer Umfangsrichtung 56 bezüglich der optischen Achse 16 benachbart zueinander angeordnet sind. Die Füße 29 können eingerichtet sein, in dem Kopplungszustand den ersten Linsenteil 2, insbesondere das Linsenbauteil 7, zu halten und somit den ersten Linsenteil 2 bezüglich des zweiten Linsenteils 3 zu zentrieren. Zum Zentrieren können die Füße 29 beispielsweise rotationssymetrisch bezüglich der optischen Achse 16 angeordnet sein. Um den ersten Linsenteil 2 zu halten, können die Füße 29 jeweils einen Radialvorsprung 30 aufweisen, der von den Füßen 29 in Richtung zu der optischen Achse 16 hin vorsteht. Der Radialvorsprung 30 kann eingerichtet sein, in dem Kopplungszustand den ersten Linsenteil 2, insbesondere eine Haptik 19 des ersten Linsenteils 2, zwischen dem Radialvorsprung 30 und dem Kapselsack 4 einzuklemmen.

### Bezugszeichenliste

1 akkommodative Intraokularlinse
2 erster Linsenteil
3 zweiter Linsenteil
4 Kapselsack
5 erste Membran
6 zweite Membran
7 Linsenbauteil
8 Optikkörper
9 Biegeelement
10 Hohlraum
11 distale Oberfläche der ersten Membran
12 proximale Oberfläche der ersten Membran
13 distale Oberfläche des Linsenbauteils
14 proximale Oberfläche des Linsenbauteils
15 Zonulafasern
16 optische Achse
17 distale Seite des ersten Linsenteils
18 proximale Seite des ersten Linsenteils
19 Haptik
21 erstes Bauteil
22 Axialdurchgangsloch des ersten Bauteils
23 zweites Bauteil
24 Axialdurchgangsloch des zweiten Bauteils
25 Zwischenraum
26 zylinderförmiger Abschnitt
27 weitere Linse
28 Radialdurchgangsloch
29 Fuß
30 Radialvorsprung
31 scheibenförmiger Abschnitt
41 distale Oberfläche des ersten Bauteils
42 proximale Oberfläche des ersten Bauteils
43 distale Oberfläche des zweiten Bauteils
44 proximale Oberfläche des zweiten Bauteils
51 Äquator des Kapselsacks
52 vorderer Bereich des Kapselsacks
53 hinterer Bereich des Kapselsacks
54 Axialrichtung
55 Radialrichtung
56 Umfangsrichtung

## Patentansprüche

1. Akkommodative Intraokularlinse zum Einbringen in den Kapselsack (4) eines Auges, mit einem ersten Linsenteil (2), der ein für Licht transparentes Linsenbauteil (7), das eine distale Oberfläche (13) des Linsenbauteils (7) und eine proximale Oberfläche (14) des Linsenbauteils (7) aufweist, eine für das Licht transparente erste Membran (5), die benachbart zu der distalen Oberfläche (13) des Linsenbauteils (7) angeordnet ist und zusammen mit dem Linsenbauteil (7) einen Hohlraum (10) begrenzt, und eine optische Achse (16) aufweist, und einem zweiten Linsenteil (3), der sich lösbar mit dem ersten Linsenteil (2) koppeln lässt, wodurch die Intraokularlinse (1) in einen Kopplungszustand bringbar ist, in dem der zweite Linsenteil (3) an einer distalen Seite (17) des ersten Linsenteils (2) angeordnet ist und eingerichtet ist, durch ein Längsverlagern des zweiten Linsenteils (3) parallel zu der optischen Achse (16) die erste Membran (5) zu verformen, wobei der zweite Linsenteil (3) ein erstes Bauteil (21), das ein distales Ende des zweiten Linsenteils (3) bildet und ein Axialdurchgangsloch (22) des ersten Bauteils (21) aufweist, und ein zweites Bauteil (23) aufweist, das eingerichtet ist, die erste Membran (5) zu verformen, und ein Axialdurchgangsloch (24) des zweiten Bauteils (23) aufweist, wobei die optische Achse (16) sich durch das Axialdurchgangsloch (22) des ersten Bauteils (21) und durch das Axialdurchgangsloch (24) des zweiten Bauteils (23) erstreckt, wobei das erste Bauteil (21) und das zweite Bauteil (23) an ihren Enden, die in dem Kopplungszustand in einer Radialrichtung (55) bezüglich der optischen Achse (16) außen angeordnet sind, fest miteinander verbunden sind, **dadurch gekennzeichnet, dass** das erste Bauteil (21) und das zweite Bauteil (23) eingerichtet sind, bei einer zunehmenden Streckung des Kapselsacks (4) zueinander hin zu verlagern und dadurch eine Rückstellkraft zu erzeugen.

2. Akkommodative Intraokularlinse zum Einbringen in den Kapselsack (4) eines Auges, mit einem ersten Linsenteil (2), der ein für Licht transparentes Linsenbauteil (7), das eine distale Oberfläche (13) des Linsenbauteils (7) und eine proximale Oberfläche (14) des Linsenbauteils (7) aufweist, eine für das Licht transparente erste Membran (5), die benachbart zu der distalen Oberfläche (13) des Linsenbauteils (7) angeordnet ist und zusammen mit dem Linsenbauteil (7) einen Hohlraum (10) begrenzt, und eine optische Achse (16) aufweist, und einem zweiten Linsenteil (3), der sich lösbar mit dem ersten Linsenteil (2) koppeln lässt, wodurch die Intraokularlinse (1) in einen Kopplungszustand bringbar ist, in dem der zweite Linsenteil (3) an einer distalen Seite (17) des ersten Linsenteils (2) angeordnet ist und eingerichtet ist, durch ein Längsverlagern des zweiten Linsenteils (3) parallel zu der optischen Achse (16) die erste Membran (5) zu verformen, wobei der zweite Linsenteil (3) ein erstes Bauteil (21), das ein distales Ende des zweiten Linsenteils (3) bildet und ein Axialdurchgangsloch (22) des ersten Bauteils (21) aufweist, und ein zweites Bauteil (23) aufweist, das sich in Axialrichtung (54) bezüglich der optischen Achse (16) mindestens bis zu dem Linsenbauteil (7) erstreckt und ein Axialdurchgangsloch (24) des zweiten Bauteils (23) aufweist, wobei die optische Achse (16) sich durch das Axialdurchgangsloch (22) des ersten Bauteils (21) und durch das Axialdurchgangsloch (24) des zweiten Bauteils (23) erstreckt, wobei das erste Bauteil (21) und das zweite Bauteil (23) an ihren Enden, die in dem Kopplungszustand in einer Radialrichtung (55) bezüglich der optischen Achse (16) außen angeordnet sind, fest miteinander verbunden sind, **dadurch gekennzeichnet, dass** das erste Bauteil (21) und das zweite Bauteil (23) eingerichtet sind, bei einer zunehmenden Streckung des Kapselsacks (4) zueinander hin zu verlagern und dadurch eine Rückstellkraft zu erzeugen.

3. Intraokularlinse gemäß Anspruch 2, wobei das erste Bauteil (21) einen zylinderförmigen Abschnitt (26) aufweist, der in dem Kopplungszustand das in der Radialrichtung (55) innenliegende Ende des ersten Bauteils (21) bildet und der den ersten Linsenteil (2) kontaktiert.

4. Intraokularlinse gemäß Anspruch 2 oder 3, wobei das zweite Bauteil (23) sich in dem Kopplungszustand in der Axialrichtung (54) mindestens bis zu einer proximalen Seite (8) des ersten Linsenteils (2) erstreckt, insbesondere wobei eine proximale Oberfläche (44) des zweiten Bauteils (23) konvex gekrümmt ausgeführt ist.

5. Intraokularlinse gemäß einem der Ansprüche 1 bis 4, wobei das erste Bauteil (21) und/oder das zweite Bauteil (23) in der Radialrichtung (55) eine maximale Erstreckung von mindestens 8,5 mm haben.

6. Intraokularlinse gemäß einem der Ansprüche 1 bis 5, wobei der Elastizitätsmodul des ersten Bauteils (21) und des zweiten Bauteils (23) von 1,5 MPa bis 5,0 MPa beträgt.

7. Intraokularlinse gemäß einem der Ansprüche 1 bis 6, wobei der zweite Linsenteil (3) eine weitere Linse (27) aufweist, insbesondere wobei die weitere Linse (27) torisch und/oder nicht akkommodativ ausgeführt ist.

8. Intraokularlinse gemäß einem der Ansprüche 1 bis 7, wobei eine distale Oberfläche (41) des ersten Bauteils (21) konvex gekrümmt ausgeführt ist.

9. Intraokularlinse gemäß einem der Ansprüche 1 bis 8, wobei das erste Bauteil (21) und/oder das zweite Bauteil (23) eine Mehrzahl an Radialdurchgangslöchern (28) aufweisen, via die eine Flüssigkeit von einer in der Radialrichtung (55) innenliegenden Seite des zweiten Linsenteils (3) zu einer in der Radialrichtung (55) außenliegenden Seite des zweiten Linsenteils (3) strömen kann.

10. Intraokularlinse gemäß einem Ansprüche 1 bis 9, wobei der zweite Linsenteil (3) eine Mehrzahl an Füßen (29) aufweist, die in einem axialen Bereich das in der Radialrichtung (55) außenliegende Ende des zweiten Linsenteils (3) bilden, in einer proximalen Richtung von dem verbliebenen zweiten Linsenteil (3) abstehen und in einer Umfangsrichtung (56) bezüglich der optischen Achse (16) benachbart zueinander angeordnet sind, insbesondere wobei die Füße (29) eingerichtet sind, den ersten Linsenteil (2), insbesondere das Linsenbauteil (7), in dem Kopplungszustand zu halten und somit den ersten Linsenteil (2) bezüglich des zweiten Linsenteils (3) zu zentrieren.

## Claims

1. Accommodative intraocular lens for insertion into the capsular bag (4) of an eye, having a first lens part (2), which comprises a light-transparent lens component (7) comprising a distal surface (13) of the lens component (7) and a proximal surface (14) of the lens component (7), a light-transparent first membrane (5) arranged adjacent to the distal surface (13) of the lens component (7) and delimiting a cavity (10) together with the lens component (7), and an optical axis (16), and a second lens part (3) which can be detachably coupled to the first lens part (2), whereby the intraocular lens (1) is able to be brought into a coupled state in which the second lens part (3) is arranged on a distal side (17) of the first lens part (2) and configured to deform the first membrane (5) by way of a longitudinal displacement of the second lens part (3) parallel to the optical axis (16), with the second lens part (3) comprising a first component (21), which forms a distal end of the second lens part (3) and has an axial through hole (22) in the first component (21), and a second component (23), which is configured to deform the first membrane (5) and has an axial through hole (24) in the second component (23), with the optical axis (16) extending through the axial through hole (22) in the first component (21) and through the axial through hole (24) in the second component (23), with the first component (21) and the second component (23) being securely connected to one another at their ends which, in the coupled state, are arranged on the outside in a radial direction (55) with respect to the optical axis (16), **characterized in that** the first component (21) and the second component (23) are configured to be displaced toward one another in the case of increased stretching of the capsular bag (4) and produce a restoring force as a result.

2. Accommodative intraocular lens for insertion into the capsular bag (4) of an eye, having a first lens part (2), which comprises a light-transparent lens component (7) comprising a distal surface (13) of the lens component (7) and a proximal surface (14) of the lens component (7), a light-transparent first membrane (5) arranged adjacent to the distal surface (13) of the lens component (7) and delimiting a cavity (10) together with the lens component (7), and an optical axis (16), and a second lens part (3) which can be detachably coupled to the first lens part (2), whereby the intraocular lens (1) is able to be brought into a coupled state in which the second lens part (3) is arranged on a distal side (17) of the first lens part (2) and configured to deform the first membrane (5) by way of a longitudinal displacement of the second lens part (3) parallel to the optical axis (16), with the second lens part (3) comprising a first component (21), which forms a distal end of the second lens part (3) and has an axial through hole (22) in the first component (21), and a second component (23), which extends at least as far as the lens component (7) in the axial direction (54) with respect to the optical axis (16) and has an axial through hole (24) in the second component (23), with the optical axis (16) extending through the axial through hole (22) in the first component (21) and through the axial through hole (24) in the second component (23), with the first component (21) and the second component (23) being securely connected to one another at their ends which, in the coupled state, are arranged on the outside in a radial direction (55) with respect to the optical axis (16), **characterized in that** the first component (21) and the second component (23) are configured to be displaced toward one another in the case of increased stretching of the capsular bag (4) and produce a restoring force as a result.

3. Intraocular lens according to Claim 2, wherein the first component (21) comprises a cylindrical portion (26) which, in the coupled state, forms the inner end of the first component (21) in the radial direction (55) and which is in contact with the first lens part (2).

4. Intraocular lens according to Claim 2 or 3, wherein the second component (23), in the coupled state, extends at least as far as a proximal side (8) of the first lens part (2) in the axial direction (54), in particular wherein a proximal surface (44) of the second component (23) has a convexly curved embodiment.

5. Intraocular lens according to any of Claims 1 to 4, wherein the first component (21) and/or the second component (23) have a maximum extent of at least 8.5 mm in the radial direction (55).

6. Intraocular lens according to any of Claims 1 to 5, wherein Young's modulus of the first component (21) and of the second component (23) is from 1.5 MPa to 5.0 MPa.

7. Intraocular lens according to any of Claims 1 to 6, wherein the second lens part (3) comprises a further lens (27), in particular wherein the further lens (27) has a toric and/or non-accommodative embodiment.

8. Intraocular lens according to any of Claims 1 to 7, wherein a distal surface (41) of the first component (21) has a convexly curved embodiment.

9. Intraocular lens according to any of Claims 1 to 8, wherein the first component (21) and/or the second component (23) have/has a plurality of radial through holes (28), through which a liquid is able to flow from an inner side of the second lens part (3) in the radial direction (55) to an outer side of the second lens part (3) in the radial direction (55).

10. Intraocular lens according to any of Claims 1 to 9, wherein the second lens part (3) comprises a plurality of feet (29) which, in an axial region, form the outer end of the second lens part (3) in the radial direction (55), protrude from the remaining second lens part (3) in a proximal direction and are arranged adjacent to one another in a circumferential direction (56) with respect to the optical axis (16), in particular wherein the feet (29) are configured to retain the first lens part (2), in particular the lens component (7), in the coupled state and consequently center the first lens part (2) in relation to the second lens part (3).

## Revendications

1. Lentille intraoculaire accommodative destinée à être insérée dans le sac capsulaire (4) d'un oeil, ladite lentille comprenant une première partie de lentille (2) qui comporte un composant de lentille (7) transparent à la lumière qui comporte une surface distale (13) du composant de lentille (7) et une surface proximale (14) du composant de lentille (7), une première membrane (5) transparente à la lumière qui est disposée de manière adjacente à la surface distale (13) du composant de lentille (7) et qui délimite une cavité (10) conjointement avec le composant de lentille (7), et qui comporte un axe optique (16), et une deuxième partie de lentille (3) qui peut être accouplée de manière amovible à la première partie de lentille (2), ce qui permet d'amener la lentille intraoculaire (1) dans un état d'accouplement dans lequel la deuxième partie de lentille (3) est disposée sur un côté distal (17) de la première partie de lentille (2) et est adaptée pour déformer la première membrane (5) par un déplacement longitudinal de la deuxième partie de lentille (3) parallèlement à l'axe optique (16), la deuxième partie de lentille (3) comportant un premier composant (21), qui forme une extrémité distale de la deuxième partie de lentille (3) et qui comporte un trou traversant axial (22) du premier composant (21), et un deuxième composant (23) qui est adapté pour déformer la première membrane (5) et qui comporte un trou traversant axial (24) du deuxième composant (23), l'axe optique (16) s'étendant à travers le trou traversant axial (22) du premier composant (21) et à travers le trou traversant axial (24) du deuxième composant (23), le premier composant (21) et le deuxième composant (23) étant solidement reliés l'un à l'autre au niveau de leurs extrémités qui, à l'état d'accouplement, sont disposées à l'extérieur par rapport à l'axe optique (16) dans une direction radiale (55), **caractérisée en ce que** le premier composant (21) et le deuxième composant (23) sont conçus pour se déplacer l'un vers l'autre à mesure que le sac capsulaire (4) s'étire de plus en plus et génèrent ainsi une force de rappel.

2. Lentille intraoculaire accommodative destinée à être insérée dans le sac capsulaire (4) d'un oeil, ladite lentille comprenant une première partie de lentille (2) qui comporte un composant de lentille (7) transparent à la lumière qui comporte une surface distale (13) du composant de lentille (7) et une surface proximale (14) du composant de lentille (7), une première membrane (5) transparente à la lumière qui est disposée de manière adjacente à la surface distale (13) du composant de lentille (7) et qui délimite une cavité (10) conjointement avec le composant de lentille (7) et comporte un axe optique (16), et une deuxième partie de lentille (3) qui peut être accouplée de manière amovible à la première partie de lentille (2), ce qui permet d'amener la lentille intraoculaire (1) dans un état d'accouplement dans lequel la deuxième partie de lentille (3) est disposée sur un côté distal (17) de la première partie de lentille (2) et est adaptée pour déformer la première membrane (5) par un déplacement longitudinal de la deuxième partie de lentille (3) parallèlement à l'axe optique (16), la deuxième partie de lentille (3) comportant un premier composant (21) qui forme une extrémité distale de la deuxième partie de lentille (3) et qui comporte un trou traversant axial (22) du premier composant (21), et un deuxième composant (23) qui s'étend dans la direction axiale (54) par rapport à l'axe optique (16) au moins jusqu'au composant de lentille (7) et qui comporte un trou traversant axial (24) du deuxième composant (23), l'axe optique (16) s'étendant à travers le trou traversant axial (22) du premier composant (21) et à travers le trou traversant axial (24) du deuxième composant (23), le premier composant (21) et le deuxième composant (23) étant solidement reliés l'un à l'autre au niveau de leurs extrémités qui, à l'état d'accouplement, sont disposées à l'extérieur par rapport à l'axe optique (16) dans une direction radiale (55), **caractérisée en ce que** le premier composant (21) et le deuxième composant (23) sont conçus pour se déplacer l'un vers l'autre à mesure que le sac capsulaire (4) s'étire de plus en plus et génèrent ainsi une force de rappel.

3. Lentille intraoculaire selon la revendication 2, le premier composant (21) comportant une portion cylindrique (26) qui, dans l'état d'accouplement, forme l'extrémité intérieur du premier composant (21) dans la direction radiale (55) et qui vient en contact avec la première partie de lentille (2).

4. Lentille intraoculaire selon la revendication 2 ou 3, le deuxième composant (23) s'étendant à l'état d'accouplement dans la direction axiale (54) au moins jusqu'à un côté proximal (8) de la première partie de lentille (2), en particulier une surface proximale (44) du deuxième composant (23) étant conçue pour être incurvée convexe.

5. Lentille intraoculaire selon l'une des revendications 1 à 4, le premier composant (21) et/ou le deuxième composant (23) présentant une extension maximale d'au moins 8,5 mm dans la direction radiale (55) .

6. Lentille intraoculaire selon l'une des revendications 1 à 5, le module d'élasticité du premier composant (21) et du deuxième composant (23) étant compris entre 1,5 MPa et 5,0 MPa.

7. Lentille intraoculaire selon l'une des revendications 1 à 6, la deuxième partie de lentille (3) comportant une autre lentille (27), en particulier l'autre lentille (27) étant conçue pour être torique et/ou non accommodative.

8. Lentille intraoculaire selon l'une des revendications 1 à 7, une surface distale (41) du premier composant (21) étant conçue pour être incurvée convexe.

9. Lentille intraoculaire selon l'une des revendications 1 à 8, le premier composant (21) et/ou le deuxième composant (23) comportant une pluralité de trous traversants radiaux (28) par le biais desquels un liquide peut s'écouler depuis un côté de la deuxième partie de lentille (3) qui est situé à l'intérieur dans la direction radiale (55) vers un côté de la deuxième partie de lentille (3) qui est situé à l'extérieur dans la direction radiale (55).

10. Lentille intraoculaire selon l'une des revendications 1 à 9, la deuxième partie de lentille (3) comportant une pluralité de pieds (29) qui, dans une zone axiale, forment l'extrémité de la deuxième partie de lentille (3) qui est située à l'extérieur dans la direction radiale (55), font saillie de la deuxième partie de lentille restante (3) dans une direction proximale et sont disposées de manière adjacente les unes aux autres par rapport à l'axe optique (16) dans une direction circonférentielle (56), en particulier les pieds (29) étant conçus pour maintenir la première partie de lentille (2), en particulier le composant de lentille (7), à l'état d'accouplement et ainsi centrer la première partie de lentille (2) par rapport à la deuxième partie de lentille (3).
